# FASCICULE DE BREVET EUROPEEN

(11) **EP 1 940 477 B1**
(45) Date de publication et mention de la délivrance du brevet: **16.05.2012**
(21) Numéro de dépôt: 05824473.2
(22) Date de dépôt: 05.12.2005
(51) Int. Cl.: A61L 2/10, A61B 8/00, A61L 2/24

(54) **APPAREIL D'IMAGERIE MEDICALE**
MEDIZINISCHES BILDGEBUNGSGERÄT
MEDICAL IMAGING APPARATUS

(30) Priorité: 26.10.2005 FR 0510958
(43) Date de publication de la demande: 09.07.2008
(73) Titulaire: Germitec, 75008 Paris (FR)
(72) Inventeur: DESHAYS, Clément, F-07120 Ruoms (FR)
(74) Mandataire: Jacobson, Claude
(86) Numéro de dépôt international: PCT/FR2005/003034
(87) Numéro de publication internationale: WO 2007/048887

(56) Documents cités:
- EP-A- 0 630 820
- EP-A- 1 532 989
- WO-A-84/00009
- WO-A-99/08137
- US-A- 4 772 795
- US-A- 5 185 532
- US-A- 5 690 113
- US-A- 6 039 928
- US-B1- 6 231 819

## Description

La présente invention concerne un appareil d'imagerie médicale.

Plus particulièrement, l'invention se rapporte à un tel appareil qui comporte un bâti sur lequel est montée au moins une unité de traitement d'informations d'imagerie médicale, raccordée au moins d'une part, à des moyens formant sonde d'imagerie et d'autre part, à des moyens formant moniteur d'affichage des images.

Un tel appareil d'imagerie médicale peut par exemple être constitué par un échographe, un endoscope ou autre. Un tel appareil est décrit dans le document US-A-5 690 113.

De très nombreux appareils d'imagerie médicale ont en effet été développés dans l'état de la technique.

Cependant, ces appareils présentent un certain nombre d'inconvénients notamment au niveau de la désinfection des sondes.

La désinfection de telles sondes est en effet aujourd'hui réalisée la plupart du temps par un simple essuyage de celles-ci, à l'aide par exemple d'un chiffon imbibé d'un produit de désinfection quelconque.

D'autres techniques de désinfection nécessitent de tremper la sonde dans une solution de désinfection appropriée.

Le problème de ces techniques de désinfection est que, soit elles sont simples à mettre en oeuvre, comme par exemple l'essuyage, mais elles ne permettent alors pas d'obtenir une désinfection optimale de la sonde, soit elles sont plus complexes à mettre en oeuvre, comme par exemple l'immersion de la sonde dans une solution de désinfection, mais elles sont alors mises en oeuvre de façon plus ou moins systématiques.

En effet, cette dernière technique nécessite de déconnecter la sonde de l'échographe, de l'immerger et de la laisser tremper dans la solution de désinfection, puis de la reconnecter à l'échographe.

Or, de telles manipulations d'une sonde peuvent entraîner des dégradations de celle-ci.

En effet, la déconnexion et la reconnexion de la sonde peuvent entraîner des dégradations des connecteurs correspondants. De même, la sonde peut également subir des dommages lors de sa désinfection dans la solution.

Or, de telles sondes sont relativement onéreuses.

Par ailleurs, ces différents techniques de désinfection imposent de disposer de moyens de désinfection correspondants, qui peuvent dans certains cas être d'une utilisation délicate voire nécessiter un confinement particulier.

Le but de l'invention est donc de résoudre ces problèmes selon la revendication 1.

A cet effet, l'invention a pour objet un appareil

Suivant d'autres caractéristiques de l'invention :
- l'enceinte est munie d'une trappe d'accès et est associée à un panneau de commande prévu sur le bâti ;
- les informations d'identification de chaque sonde se présentent sous la forme d'un code à barre et les moyens d'acquisition correspondants (16) de l'enceinte comprennent un lecteur de code ;
- l'enceinte comporte une potence de suspension de la sonde et le lecteur est fixé sur cette potence ;
- les moyens d'acquisition d'informations de caractérisation du cycle de désinfection comprennent des moyens d'acquisition d'informations choisies dans le groupe d'informations comprenant des informations d'identification de l'enceinte et/ou de l'appareil et des informations d'horodatage du cycle de désinfection ;
- l'enceinte comporte des moyens de génération d'un rayonnement UV de désinfection de la sonde et les informations de caractérisation du cycle de désinfection comprennent des informations de dose d'UV émise lors du cycle, issues d'un capteur correspondant implanté dans l'enceinte.
- le capteur d'UV est implanté sous la potence de l'enceinte ;
- les moyens d'association des informations sont associés à des moyens de visualisation de celles-ci, de stockage de celles-ci et/ou d'impression de celles-ci ;
- les moyens d'association des informations sont adaptés pour n'émettre les informations de traçabilité que si la sonde correspondante a bien été identifiée lors de sa mise en place et de son retrait de l'enceinte avant et après le cycle de désinfection, respectivement ;
- l'enceinte présente une surface intérieure de section circulaire ;
- la surface intérieure de l'enceinte présente des décrochements semi-circulaires de réception des moyens de génération du rayonnement ;
- les moyens de génération du rayonnement comportent quatre tubes disposés à 90° les uns des autres dans l'enceinte ;
- l'enceinte présente une surface intérieure revêtue au moins en partie par du polytétrafluoroéthylène ;
- l'enceinte comporte des moyens de suspension d'au moins une sonde dans l'enceinte et des moyens de mise en rotation de la sonde dans l'enceinte ;
- les moyens de mise en rotation sont adaptés pour provoquer un déplacement oscillant sur une plage angulaire prédéterminée des moyens de suspension ;
- les moyens de mise en rotation comprennent des moyens de motorisation électrique associés à un crochet de suspension de la sonde dans l'enceinte ;
- les moyens de suspension et les moyens de motorisation sont associés à la potence s'étendant dans l'enceinte ;
- l'enceinte comporte des moyens de suspension d'au moins une sonde dans l'enceinte et des moyens de génération dans l'enceinte, d'un rayonnement de désinfection et des moyens, transparents au rayonnement, de maintien de la sonde dans un état allongé et une orientation sensiblement verticale, sous les moyens de suspension ;
- les moyens de maintien sont adaptés pour maintenir la sonde sensiblement au centre de l'enceinte ;
- les moyens de maintien comprennent des moyens de guidage de la sonde dans l'enceinte ;
- les moyens de guidage comprennent un ou plusieurs organes en forme de peigne de guidage de la sonde répartis sur la hauteur de l'enceinte ;
- les moyens de guidage comprennent un ou plusieurs tubes de réception de la sonde ;
- les moyens de maintien comprennent un poids adapté pour être accroché à l'extrémité libre de la sonde ; et
- les moyens de maintien sont réalisés en oxyde de silicium. L'invention sera mieux comprise à la lecture de la description qui va suivre, donnée uniquement à titre d'exemple et faite en se référant aux dessins annexés, sur lesquels :
- la Fig.1 représente une vue en perspective d'un exemple de réalisation d'un appareil d'imagerie médicale suivant l'invention ;
- la Fig.2 représente une vue de détail en perspective d'un exemple de réalisation d'une enceinte de désinfection entrant dans la constitution d'un appareil selon l'invention ;
- la Fig.3 représente un schéma synoptique illustrant la structure et le fonctionnement de moyens de traçabilité de la désinfection d'une sonde, entrant dans la constitution d'un appareil selon l'invention ;
- la Fig.4 représente une vue de dessus en coupe d'une portion d'enceinte entrant dans la constitution d'un appareil selon l'invention et
- les Fig.5 et 6 représentent des détails de réalisation de cette enceinte.

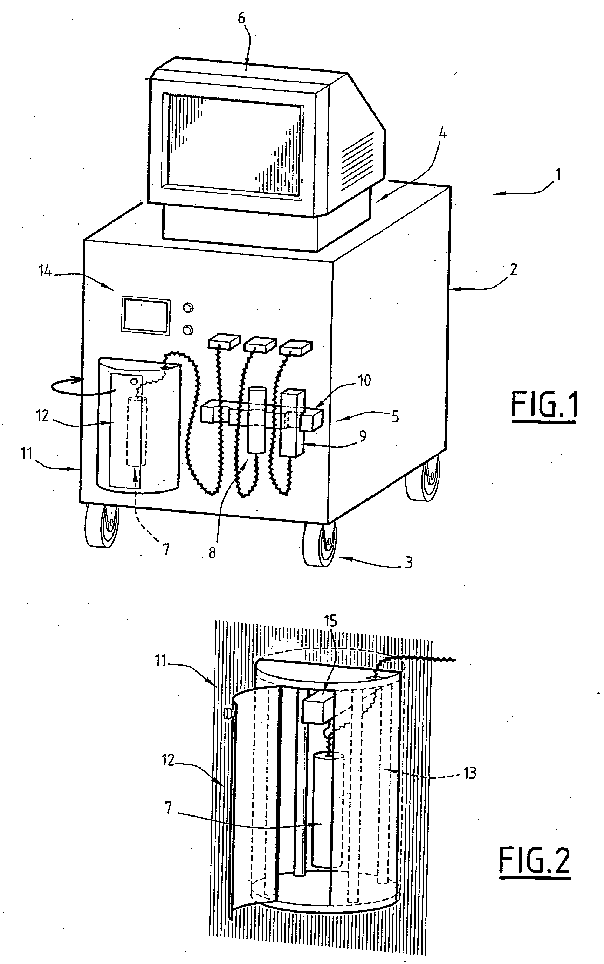

On a en effet illustré sur les figures 1 et 2, un exemple de réalisation d'un appareil d'imagerie médicale.

Dans l'exemple de réalisation illustré sur ces figures, cet appareil est constitué par exemple par un échographe qui est désigné par la référence générale 1 sur la figure 1

Cet appareil comporte alors un bâti désigné par la référence générale 2, monté par exemple sur des roulettes dont l'une est désignée par la référence générale 3, pour être déplaçable.

Sur ce bâti sont montés différents organes de l'échographe, tels que par exemple une unité de traitement d'informations d'imagerie médicale désignée par la référence générale 4, raccordée au moins d'une part, à des moyens formant sonde d'imagerie désignés par la référence générale 5 sur cette figure, et d'autre part, à des moyens formant moniteur d'afficheur de ces images désignés par la référence générale 6.

Il va de soi bien entendu que d'autres organes et composants peuvent être prévus tels que par exemple des moyens d'impression, etc.

Dans l'exemple de réalisation illustré sur cette figure 1, le bâti comporte également des moyens de rangement des sondes.

Les sondes sont par exemple au nombre de trois et sont désignées par les références générales 7, 8 et 9 sur cette figure et comportent en fait, une partie active de sonde proprement dite raccordée par un câble de liaison à un connecteur respectif permettant de raccorder la sonde au reste de l'appareil et en particulier à l'unité de traitement d'informations de celui-ci.

Les moyens de rangement des sondes sont par exemple désignés par la référence générale 10 sur cette figure et comportent tout organe approprié de rangement de sonde, tel que par exemple un organe en forme de peigne d'accrochage comme cela est illustré.

Il va de soi bien entendu que d'autres modes de réalisation encore peuvent être envisagés.

Selon l'invention, cet appareil d'imagerie médicale comporte également au moins une enceinte intégrée de désinfection des moyens formant sonde.

Une telle enceinte est par exemple désignée par la référence générale 11 sur la figure 1.

En fait, et comme cela est illustré sur les figures 1 et 2, une telle enceinte 11 est intégrée dans le bâti de l'appareil et comporte par exemple une trappe d'accès désignée par la référence générale 12 sur ces figures, permettant à un opérateur d'introduire une sonde telle que par exemple la sonde 7 dans cette enceinte, afin d'y être soumise à un cycle de désinfection.

De très nombreux modes de réalisation de la trappe d'accès peuvent être envisagés. Celle-ci peut par exemple être articulée sur le bâti pour permettre à un opérateur d'ouvrir et de fermer l'enceinte.

L'enceinte 11 comporte alors des moyens de génération d'un rayonnement de désinfection de la sonde, ce rayonnement étant par exemple un rayonnement UV de type C par exemple.

Dans ce cas, ces moyens de génération comprennent par exemple des tubes UVC tels que désignés par la référence générale 13 sur cette figure 2.

Un exemple de réalisation plus détaillé de ces tubes et de leur disposition sera donné par la suite.

Le fonctionnement de cette enceinte est alors contrôlé par des moyens de pilotage associés par exemple à un panneau de commande désigné par la référence générale 14 sur cette figure, et prévu sur le bâti 2 de l'appareil.

De plus, l'enceinte est également munie de moyens de suspension de la sonde dans celle-ci.

Ces moyens de suspension sont par exemple formés par une potence désignée par la référence générale 15, permettant à l'opérateur d'accrocher le câble 7 de la sonde, afin de maintenir la sonde en position suspendue dans l'enceinte en vue d'optimiser sa désinfection.

Le document EP-A-0 839 537 décrit déjà un dispositif de ce type pour le support d'instruments dans une enceinte notamment de décontamination et une enceinte correspondante.

On conçoit alors que l'intégration d'une telle enceinte dans un appareil d'imagerie médicale permet de faciliter grandement les opérations de désinfection des sondes dans la mesure où la désinfection par rayonnement UV est extrêmement efficace et où il n'est plus nécessaire de déconnecter/reconnecter la sonde de l'appareil et de la faire tremper dans une solution de désinfection pour obtenir une désinfection efficace.

Le passage de la sonde pendant quelques minutes dans l'enceinte de désinfection permet en effet d'obtenir une désinfection optimale de celle-ci et ce, de manière très simple pour l'opérateur, par exemple entre deux patients.

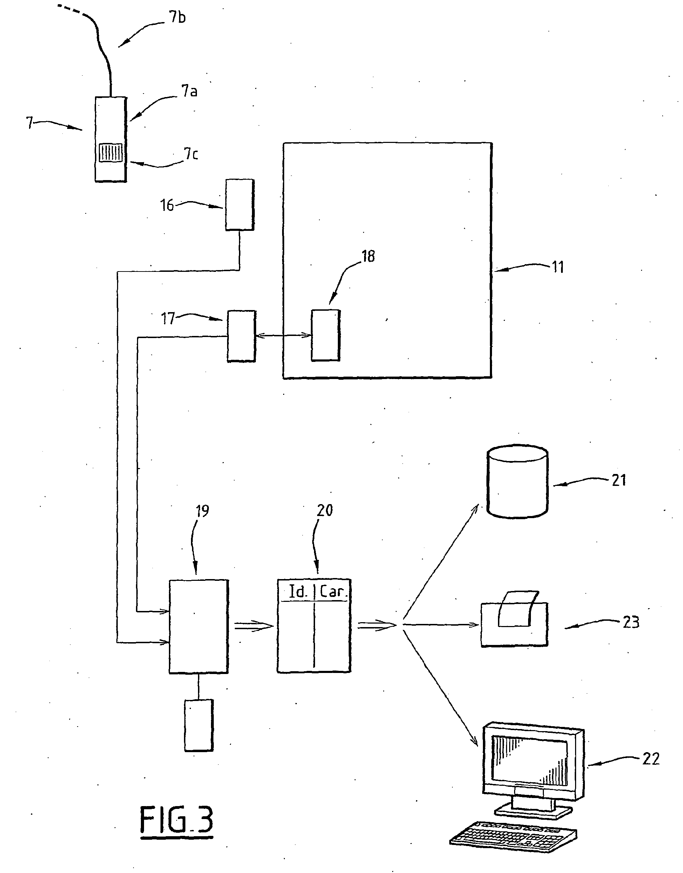

De plus, et comme cela est illustré sur la figure 3, des moyens de traçabilité de l'opération de désinfection des sondes peuvent être envisagés.

On reconnaît en effet sur cette figure 3, l'enceinte de désinfection 11 et la sonde désignée de façon générale par la référence 7.

En fait, et comme cela a été expliqué précédemment, une telle sonde comporte de façon générale, une partie active désignée par la référence générale 7a sur cette figure et un câble de raccordement désigné par la référence générale 7b.

Cette sonde est alors adaptée pour être mise en place et retirée de l'enceinte de désinfection 11, cette enceinte de désinfection étant adaptée pour mettre en oeuvre un cycle de désinfection de cette sonde, par rayonnement de désinfection.

On notera à cet égard qu'un passe-câble est prévu, par exemple au niveau de la trappe d'accès ou autres, pour permettre d'introduire la sonde dans l'enceinte sans déconnecter celle-ci du reste de l'appareil.

Dans l'appareil selon l'invention, chaque sonde porte des informations d'identification de celle-ci.

A titre d'exemple, ces informations d'identification peuvent être constituées par un code à barre désigné par la référence générale 7c sur la figure 3, ce code à barre étant par exemple porté par la partie active ou encore par le câble de raccordement de la sonde.

Bien entendu, d'autres modes de réalisation peuvent être envisagés.

L'enceinte est alors associée à des moyens d'acquisition de ces informations d'identification de chaque sonde.

Ces moyens d'acquisition de ces informations d'identification sont désignés par la référence générale 16 sur cette figure 3 et comprennent par exemple tout capteur approprié tel que par exemple un lecteur de code à barre ou autre.

Ce capteur est alors adapté pour acquérir les informations d'identification de la ou de chaque sonde lors de sa mise en place et de son retrait de l'enceinte au début et à la fin d'un cycle de désinfection.

A titre d'exemple, ces moyens d'acquisition peuvent se présenter sous la forme d'un capteur extérieur à l'enceinte se présentant par exemple sous la forme d'un capteur de type « douchette » ou encore sous la forme d'un capteur directement intégré dans l'enceinte de désinfection par exemple sur la potence 15 de cette enceinte.

De plus, l'enceinte est associée à des moyens d'acquisition d'informations de caractérisation du cycle de désinfection, c'est-à-dire plus particulièrement des conditions de son déroulement.

Ces moyens sont désignés par la référence générale 17 sur cette figure et peuvent comporter différents types de moyens d'acquisition d'informations adaptés pour acquérir des informations choisies dans un groupe d'informations comprenant par exemple des information d'identification de l'enceinte et/ou de l'appareil, chaque enceinte/appareil étant alors affecté d'un numéro d'identification spécifique stocké dans celle-ci, des information d'horodatage du cycle permettant par exemple d'acquérir la date du cycle, le numéro journalier du cycle, l'heure de début et l'heure de fin du cycle, à partir d'un circuit formant horloge, etc.

Ces informations de caractérisation peuvent également comporter des informations relatives à la dose d'UV émise lors d'un cycle si l'enceinte est une enceinte de désinfection munie de moyens de génération de rayonnement UV de désinfection.

Ces informations peuvent alors être déterminées à partir d'un capteur de tout type approprié déjà connu dans l'état de la technique et désigné par exemple par la référence générale 18 sur cette figure.

Ce capteur peut alors être par exemple implanté sous la potence 15 de l'enceinte.

Ces différentes informations, c'est-à-dire les informations d'identification de la ou de chaque sonde et les informations de caractérisation du cycle de désinfection, sont alors transmises à une unité de traitement d'informations désignée par la référence générale 19 sur cette figure et constituée par tout calculateur approprié, par exemple intégré dans les moyens de pilotage du fonctionnement de l'enceinte, pour mettre en oeuvre une fonction d'association de ces informations afin d'engendrer des informations de traçabilité de la désinfection.

En effet, cette unité de traitement d'information 19 est adaptée pour associer les informations d'identification de la ou de chaque sonde présente dans l'enceinte lors d'un cycle de désinfection avec les informations de caractérisation du déroulement de ce cycle, afin de délivrer des informations permettant d'assurer la traçabilité de la désinfection de la ou de chaque sonde.

Ces informations de traçabilité sont désignées par la référence générale 20 sur la figure et permettent donc de mettre en relation chaque sonde avec les conditions dans lesquelles s'est déroulé le cycle de désinfection correspondant.

Il est à noter que ces informations de traçabilité ne peuvent être émises que si une sonde a bien été identifiée lors de son introduction dans l'enceinte avant le début du cycle et lors de son retrait de cette enceinte après la fin de ce cycle.

L'opérateur doit donc alors obligatoirement identifier la sonde lors de sa mise en place et de son retrait de l'enceinte. Dans le cas contraire, l'unité de traitement d'informations n'engendre pas les informations de traçabilité.

Ces informations de traçabilité sont alors disponibles pour assurer la traçabilité de l'opération de désinfection en vue par exemple d'un stockage de ces informations dans des moyens de stockage d'informations comme cela est illustré en 21 sur cette figure, d'une visualisation de celles-ci par exemple sur un afficheur quelconque désigné par la référence générale 22, ou encore d'une impression de celles-ci par exemple à l'aide de moyens d'impression quelconques telle qu'une imprimante désignée par la référence générale 23.

On notera par exemple qu'une telle imprimante peut être adaptée pour imprimer les informations de traçabilité sur un autocollant pouvant être associé par exemple à un dossier de patient ayant été en contact avec la sonde désinfectée, un registre de traçabilité, etc...

A titre d'exemple, les informations de caractérisation portées par cet autocollant comportent alors une information de dose d'UV reçue par la sonde lors de son passage dans l'enceinte de désinfection, cette dose étant par exemple déterminée à partir de la puissance ou éclairement UV émis lors du cycle multiplié par la durée de ce cycle.

On sait en effet que ce paramètre peut être déterminant pour obtenir tel ou tel niveau de désinfection des sondes.

On conçoit alors qu'un tel système permet d'assurer une traçabilité optimale de la désinfection des sondes de ce type dans la mesure où les informations de traçabilité permettent de garantir le passage de la sonde dans l'enceinte et de vérifier les informations de caractérisation du cycle de désinfection subi par la sonde, c'est-à-dire notamment le moment où cette désinfection a eu lieu, l'enceinte dans laquelle s'est déroulé le cycle de désinfection et la dose notamment d'UV reçue par la sonde.

Bien entendu d'autres modes de réalisation peuvent encore être envisagés.

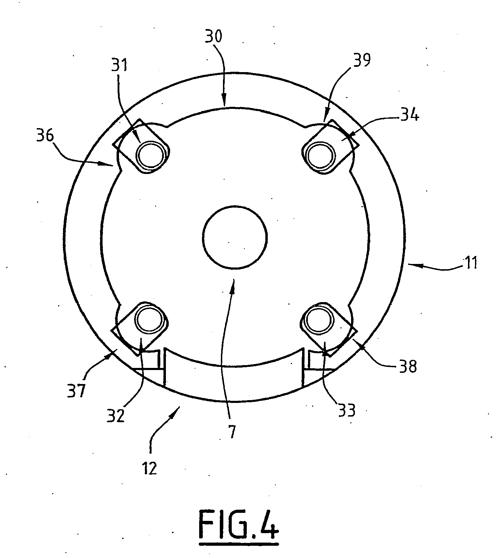

Sur la figure 4, on a illustré un exemple de réalisation d'une telle enceinte de désinfection.

Celle-ci est toujours désignée par la référence générale 11 sur cette figure 4 et la trappe d'accès à celle-ci est désignée par la référence générale 12.

La sonde est toujours désignée par la référence générale 7.

Comme cela est illustré, l'enceinte présente alors par exemple une surface intérieure de section circulaire.

Cette surface est désignée par la référence générale 30 sur cette figure et permet alors d'optimiser la réflexion du rayonnement engendré par les moyens de génération de rayonnement sur la sonde à désinfecter.

Dans l'exemple de réalisation illustré sur cette figure 4, ces moyens de génération comprennent en fait quatre tubes désignés respectivement par les références générales 31, 32, 33 et 34, répartis par exemple à 90° les uns des autres dans des décrochements semi-circulaires de réception correspondants ménagés dans cette surface intérieure de l'enceinte.

Ces décrochements semi-circulaires sont désignés respectivement par les références 36, 37, 38 et 39 sur cette figure.

Il va de soi bien entendu qu'un nombre différent de tubes et une disposition autre de ceux-ci peuvent également être envisagés.

De plus, l'enceinte peut également être optimisée par exemple si la surface intérieure 30 est revêtue au moins en partie et de préférence en totalité par du polytétrafluoroéthylène (PTFE) également connu sous la marque TE-FLON. En effet, il a été constaté que l'utilisation d'un tel matériau de revêtement de la surface permettait également d'optimiser encore la réflexion du rayonnement et donc la qualité de la désinfection.

On conçoit alors qu'une telle structure et en particulier l'utilisation d'une telle surface intérieure pour l'enceinte permettent d'optimiser la réflexion des rayonnements émis par les moyens de génération de ce rayonnement afin d'optimiser la désinfection de la sonde.

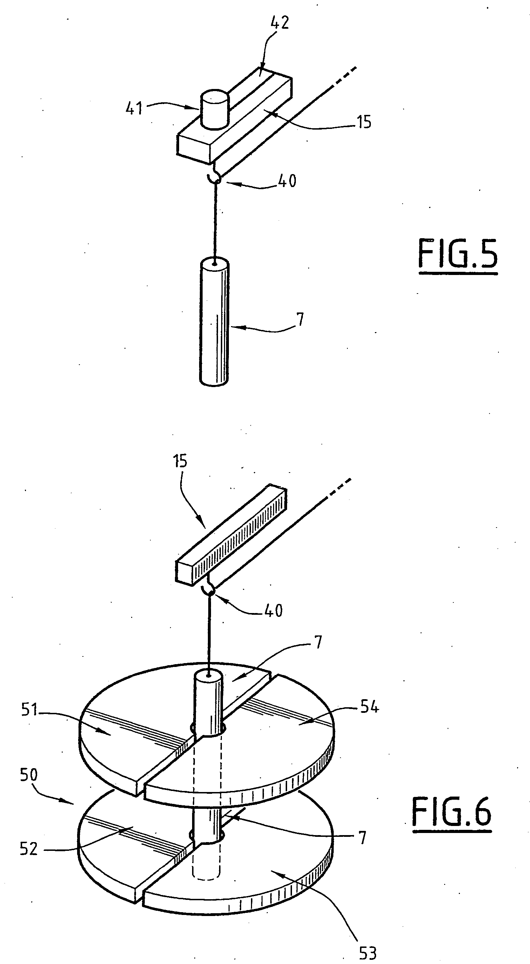

On a illustré sur la figure 5, un perfectionnement d'une telle enceinte dans lequel la potence 15 de suspension de la sonde 7 comporte des moyens de mise en rotation de la sonde dans l'enceinte.

En fait, cette potence 15 entre dans la constitution de moyens de suspension d'au moins une sonde à désinfecter, dans l'enceinte.

De manière avantageuse, ces moyens de suspension peuvent par exemple être réglables en hauteur dans l'enceinte par tout mécanisme de réglage connu dans l'état de la technique, comme par exemple un mécanisme à vis-écrou manoeuvrable grâce à une molette par l'opérateur.

Bien entendu, d'autres modes de réalisation peuvent être envisagés.

Pour résoudre des problèmes d'homogénéité de la désinfection dans l'enceinte, les moyens de suspension de la sonde comprennent des moyens de mise en rotation de cette sonde dans l'enceinte, lors de sa désinfection.

A titre d'exemple, une rotation complète sur eux-mêmes ou encore un déplacement oscillant sur une plage angulaire prédéterminée de ces moyens de suspension et donc de la sonde, peuvent être envisagés.

Dans l'exemple de réalisation illustré sur la figure 5, les moyens de suspension comprennent en fait un organe en forme de crochet désigné par la référence générale 40 auquel peut être suspendue la sonde désignée par la référence générale 7, ce crochet étant par exemple monté déplaçable à rotation par rapport à la potence 15 sous l'action de moyens de motorisation désignés par la référence générale 41 et comportant par exemple un moteur électrique associé à des moyens de réduction de vitesse ou autre, dont l'arbre de sortie est associé de toute manière appropriée au crochet 40.

Ce moteur peut par exemple être placé sur la potence 15 au droit du crochet.

L'alimentation de ce moteur est alors assurée par des conducteurs électriques désignés par la référence générale 42 sur cette figure permettant de raccorder ces moyens de motorisation à une unité de commande du fonctionnement de l'enceinte, implantée dans l'appareil par exemple.

Lors du fonctionnement de l'enceinte, les moyens de motorisation sont alors pilotés de façon à provoquer le déplacement des moyens de suspension et donc de la sonde afin d'homogénéiser l'exposition de celle-ci par exemple au rayonnement UV de désinfection.

Il va de soi bien entendu que d'autres modes de réalisation encore peuvent être envisagés, en particulier de ces moyens de suspension et de ces moyens de motorisation.

La figure 6 illustre encore un autre perfectionnement de cette enceinte.

Comme cela a été indiqué précédemment, une telle enceinte comporte la potence désignée par la référence générale 15 qui s'étend dans celle-ci pour entrer dans la constitution des moyens de suspension de la sonde à désinfecter dans l'enceinte.

Pour améliorer encore la qualité de la désinfection dans l'enceinte selon l'invention, il est prévu des moyens, transparents au rayonnement, de maintien de la sonde dans un état allongé et une orientation sensiblement verticale, sous les moyens de suspension.

Ceci permet par exemple d'éviter toute déformation ou entortillement de la sonde qui pourrait se traduire par une disposition et/ou un état de celui-ci défavorables à sa désinfection.

Différents modes de réalisation de ces moyens de maintien de l'instrument peuvent être envisagés.

En fait, ces moyens de maintien sont adaptés pour maintenir de préférence la sonde sensiblement au centre de l'enceinte comme cela est illustré.

Ces moyens de maintien peuvent alors comporter par exemple des moyens de guidage de la sonde comme ceux illustrés sur cette figure et désignés par la référence générale 50.

En effet, dans l'exemple de réalisation illustré sur la figure 6, ces moyens de maintien comprennent un ou plusieurs organes en forme de peigne de guidage répartis sur la hauteur de l'enceinte, pour maintenir l'instrument en position optimale de désinfection.

Dans l'exemple illustré, deux séries de deux peignes complémentaires désignés respectivement par les références 51, 52, 53 et 54 sont utilisés à deux hauteurs différentes dans l'enceinte pour maintenir la sonde dans la position optimale.

Il va de soi bien entendu que d'autres modes de réalisation de ces moyens peuvent être envisagés. Ainsi et à titre d'exemple un ou plusieurs tubes de réception d'une ou de plusieurs sondes peuvent également être envisagés, ces tubes s'étendant par exemple au voisinage du centre de l'enceinte dans une position verticale.

Cependant, et selon un autre mode de réalisation encore, ces moyens de maintien peuvent également être formés par un poids adapté pour être accroché et/ou suspendu à l'extrémité libre correspondante de la sonde afin de maintenir celle-ci en position.

Bien entendu et comme cela a été indiqué précédemment, ces moyens de maintien sont réalisés en matériau transparent au rayonnement tel que par exemple à un rayonnement UV de type C.

Dans ce cas, ces moyens de maintien sont réalisés à titre d'exemple en oxyde de silicium.

Ce maintien en position de là sonde permet alors d'assurer qu'elle est dans la position la plus favorable possible à sa désinfection dans l'enceinte.

On conçoit alors qu'un tel appareil présente un certain nombre d'avantages par rapport aux appareils de l'état de la technique, notamment en ce qui concerne la désinfection des sondes.

Celle-ci peut en effet être mise en oeuvre très simplement et très rapidement par un opérateur, pour obtenir une désinfection optimale des sondes.

## Revendications

1. Appareil d'imagerie médicale, du type comportant un bâti (2) sur lequel est montée au moins une unité de traitement d'informations d'imagerie médicale (4) raccordée au moins d'une part, à des moyens formant sonde d'imagerie (5, 7, 8, 9), et d'autre part, à des moyens formant moniteur d'affichage des images (6), **caractérisé en ce que** chaque le bâti (2) comporte au moins une enceinte intégrée (11) de désinfection des moyens formant sonde, comportant des moyens (13) de génération d'un rayonnement de désinfection et adaptée pour mettre en oeuvre un cycle de désinfection de ceux-ci, et **en ce que** chaque sonde (7) comporte des informations d'identfication (7c) et **en ce que** l'enceinte (11) est associée à des moyens (16) d'acquisition des informations d'identificafion de la ou de chaque sonde (7) lors de sa mise en place et de son retrait de l'enceinte (11) au début et à la fin dun cycle de désinfection, à des moyens (17, 18) d'acquisition d'informations de caractérisation du cycle de désinfection et à des moyens (19, 20 21, 22, 23) d'association des informations d'identification de la ou de chaque sonde et des informations de caractérisation du cycle de désinfection pour engendrer des informations de traçabilité de la désinfection de la ou de chaque sonde..

2. Appareil selon la revendication 1, **caractérisé en ce que** l'enceinte est munie d'une trappe d'accès (12) et est associée à un panneau de commande (14) prévu sur le bâti (2).

3. Appareil selon la revendication 1 ou 2, **caractérisé en ce que** les informations d'identification (7c) de chaque sonde se présentent sous la forme d'un code à barre et **en ce que** les moyens d'acquisition correspondants (16) de l'enceinte (11) comprennent un lecteur de code.

4. Appareil selon la revendication3, **caractérisé en ce que** l'enceinte (11) comporte une potence de suspension (15) de la sonde et **en ce que** le lecteur (16) est fixé sur cette potence.

5. Appareil selon l'une quelconque des revendications 1 à4, **caractérisé en ce que** les moyens (17, 18) d'acquisition d'informations de caractérisation du cycle de désinfection comprennent des moyens d'acquisition d'informations choisies dans le groupe d'informations comprenant des informations d'identification de l'enceinte (11) et/ou de l'appareil et des informations d'horodatage du cycle de désinfection.

6. Appareil selon l'une quelconque des revendications 1 à 5, **caractérisé en ce que** l'enceinte (11) comporte des moyens de génération d'un rayonnement UV de désinfection de la sonde et **en ce que** les informations de caractérisation du cycle de désinfection comprennent des informations de dose d'UV émise lors du cycle, issues d'un capteur correspondant (18) implanté dans l'enceinte.

7. Appareil selon les revendications 4 ou 5 et6, **caractérisé en ce que** le capteur d'UV (18) est implanté sous la potence de l'enceinte.

8. Appareil selon l'une quelconque des revendications 1 à 7, **caractérisé en ce que** les moyens (19) d'association des informations sont associés à des moyens de visualisation (22) de celles-ci, de stockage (21) de celles-ci et / ou d'impression (23) de celles-ci.

9. Appareil selon l'une quelconque des revendications 1 à 8, **caractérisé en ce que** les moyens (19) d'association des informations sont adaptés pour n'émettre les informations de traçabilité que si la sonde correspondante (7) a bien été identifiée lors de sa mise en place et de son retrait de l'enceinte (11) avant et après le cycle de désinfection, respectivement.

10. Appareil selon l'une quelconque des revendications précédentes, **caractérisé en ce que** l'enceinte (11) présente une surface intérieure (30) de section circulaire.

11. Appareil selon la revendication 10, **caractérisé en ce que** la surface intérieure (30) de l'enceinte présente des décrochements semi-circulaires (36, 37, 38, 39) de réception des moyens (31, 32, 33, 34) de génération du rayonnement.

12. Appareil selon la revendication11, **caractérisé en ce que** les moyens de génération du rayonnement comportent quatre tubes disposés à 90° les uns des autres dans l'enceinte.

13. Appareil selon l'une quelconque des revendications précédentes, **caractérisé en ce que** l'enceinte (11) présente une surface intérieure revêtue au moins en partie par du polytétrafluoroéthylène (PTFE).

14. Appareil selon l'une quelconque des revendications précédentes, **caractérisé en ce que** l'enceinte (11) comporte des moyens de suspension d'au moins une sonde dans l'enceinte et des moyens (40, 41) de mise en rotation de la sonde (7) dans l'enceinte.

15. Appareil selon la revendication14, **caractérisé en ce que** les moyens de mise en rotation sont adaptés pour provoquer un déplacement oscillant sur une plage angulaire prédéterminée des moyens de suspension.

16. Appareil selon la revendication 14 ou 15, **caractérisé en ce que** les moyens de mise en rotation comprennent des moyens de motorisation électrique (41) associés à un crochet (40) de suspension de la sonde (7) dans l'enceinte.

17. Appareil selon les revendications 4 et16, **caractérisé en ce que** les moyens de suspension et les moyens de motorisation sont associés à la potence (15) s'étendant dans l'enceinte.

18. Appareil selon l'une quelconque des revendications précédentes, **caractérisé en ce que** l'enceinte comporte des moyens (15) de suspension d'au moins une sonde (7) dans l'enceinte et des moyens de génération dans l'enceinte, d'un rayonnement de désinfection et des moyens (50), transparents au rayonnement, de maintien de la sonde (7) dans un état allongé et une orientation sensiblement verticale, sous les moyens de suspension (15).

19. Appareil selon la revendication18, **caractérisé en ce que** les moyens de maintien sont adaptés pour maintenir la sonde (7) sensiblement au centre de l'enceinte (11).

20. Appareil selon la revendication 18 ou 19, **caractérisé en ce que** les moyens de maintien comprennent des moyens de guidage (51, 52, 53, 54) de la sonde dans l'enceinte.

21. Appareil selon la revendication20, **caractérisé en ce que** les moyens de guidage comprennent un ou plusieurs organes (51, 52, 53, 54) en forme de peigne de guidage de la sonde répartis sur la hauteur de l'enceinte (11).

22. Appareil selon la revendication 21 **caractérisé en ce que** les moyens de guidage comprennent un ou plusieurs tubes de réception de la sonde (7).

23. Appareil selon l'une quelconque des revendications 18 à 22, **caractérisé en ce que** les moyens de maintien comprennent un poids adapté pour être accroché à l'extrémité libre de la sonde (7).

24. Appareil selon l'une quelconque des revendications 18 à 23, **caractérisé en ce que** les moyens de maintien sont réalisés en oxyde de silicium.

## Claims

1. Medical imaging apparatus of the type comprising a frame (2) on which is mounted at least one medical imaging data processing unit (4) connected at least on the one hand to imaging sensor forming means (5, 7, 8, 9) and on the other hand to image display forming means (6), **characterized in that** the frame (2) comprises at least one integrated chamber (11) for disinfecting the sensor forming means, comprising means (13) for generating a disinfecting radiation and adapted to implement a disinfecting cycle thereof, and **in that** each sensor (7) comprises identification data (7c), and **in that** the chamber (11) is associated with means (16) for acquiring the identification data of the or of each sensor (7) when it is put in place in and removed from the chamber (11) at the start and at the end of a disinfecting cycle, with means (17, 18) for acquiring data characterizing the disinfecting cycle and with means (19, 20, 21, 22, 23) for associating the identification data of the or of each sensor and data characterizing the disinfecting cycle to generate traceability data of the disinfection of the or of each sensor.

2. Apparatus according to claim 1, **characterized in that** the chamber is equipped with an access hatch (12) and is associated with a control panel (14) provided on the frame (2).

3. Apparatus according to claim 1 or 2, **characterized in that** the identification data (7c) of each sensor are in the form of a bar code, and **in that** the corresponding acquisition means (16) of the chamber (11) comprise a code reader.

4. Apparatus according to claim 3, **characterized in that** the chamber (11) comprises a suspension boom (15) for the sensor, and **in that** the reader (16) is fixed to this boom.

5. Apparatus according to any one of claims 1 to 4, **characterized in that** the means (17, 18) for acquiring data characterizing the disinfecting cycle comprise means for acquiring data chosen from the group of data comprising identification data of the chamber (11) and/or of the apparatus and time and date data of the disinfecting cycle.

6. Apparatus according to any one of claims 1 to 5, **characterized in that** the chamber (11) comprises means for generation of UV radiation for disinfecting the sensor and **in that** the data characterizing the disinfecting cycle comprise data on the dose of UV emitted during the cycle, provided by a corresponding sensing element (18) installed in the chamber.

7. Apparatus according to claims 4 or 5 and 6, **characterized in that** the UV sensing element (18) is installed under the boom of the chamber.

8. Apparatus according to any one of claims 1 to 7, **characterized in that** the means (19) for association of the data are associated with means (22) for visualizing these, means (21) for storage of these and/or means (23) for printing of these.

9. Apparatus according to any one of claims 1 to 8, **characterized in that** the means (19) for associating data are adapted to emit traceability data only if the corresponding sensor (7) has been properly identified when it is put in place in and removed from the chamber (11) before and, respectively, after the disinfecting cycle.

10. Apparatus according to any one of the preceding claims, **characterized in that** the chamber (11) has an internal surface (30) of circular cross section.

11. Apparatus according to claim 10, **characterized in that** the internal surface (30) of the chamber has semicircular recesses (36, 37, 38, 39) for receiving means (31, 32, 33, 34) for generating radiation.

12. Apparatus according to claim 11, **characterized in that** the means for generating radiation comprise four tubes disposed at 90° with respect to one another in the chamber.

13. Apparatus according to any one of the preceding claims, **characterized in that** the chamber (11) has an internal surface coated at least in part by polytetrafluoroethylene (PTFE).

14. Apparatus according to any one of the preceding claims, **characterized in that** the chamber (11) comprises means for suspending at least one sensor in the chamber and means (40, 41) for rotating the sensor (7) in the chamber.

15. Apparatus according to claim 14, **characterized in that** the rotation means are adapted to cause an oscillating displacement over a predetermined angular range of the suspension means.

16. Apparatus according to claim 14 or 15, **characterized in that** the rotation means comprise electric drive means (41) associated with a hook (40) for suspending the sensor (7) in the chamber.

17. Apparatus according to claims 4 and 16, **characterized in that** the suspension means and the drive means are associated with the boom (15) extending into the chamber.

18. Apparatus according to any one of the preceding claims, **characterized in that** the chamber comprises means (15) for suspending at least one sensor (7) in the chamber and means for generating disinfecting radiation in the chamber and means (50) transparent to the radiation for holding the sensor (7) in an elongated state and a substantially vertical orientation under the suspension means (15).

19. Apparatus according to claim 18, **characterized in that** the holding means are adapted to hold the sensor (7) substantially at the centre of the chamber (11).

20. Apparatus according to claim 18 or 19, **characterized in that** the holding means comprise means (51, 52, 53, 54) for guiding the sensor in the chamber.

21. Apparatus according to claim 20, **characterized in that** the guiding means comprise one or more members (51, 52, 53, 54) in comb form for guiding the sensor distributed over the height of the chamber (11).

22. Apparatus according to claim 21, **characterized in that** the guiding means comprise one or more tubes for receiving the sensor (7).

23. Apparatus according to any one of claims 18 to 22, **characterized in that** the holding means comprise a weight adapted to be coupled to the free end of the sensor (7).

24. Apparatus according to any one of claims 18 to 23, **characterized in that** the holding means are made of silicon oxide.

## Patentansprüche

1. Vorrichtung für die medizinische Bildgebung von dem Typ, der ein Gestell (2) aufweist, an dem mindestens eine Einheit (4) zum Verarbeiten von medizinischen Bildgebungsinformationen montiert ist, die zumindest einerseits mit Einrichtungen (5, 7, 8, 9), die eine Bildgebungssonde darstellen, und andererseits mit Einrichtungen (6) verbunden ist, die eine Kontrollbildapparatur zum Anzeigen der Bilder darstellen,
**dadurch gekennzeichnet,**
**dass** das Gestell (2)zumindestens eine integrierte Kammer (11) für die Desinfektion der eine Bildgebungssonde darstellenden Einrichtungen aufweist, welche Einrichtungen (13) zum Erzeugen einer Desinfektionsstrahlung aufweist, die dazu ausgelegt sind, einen Zyklus zu deren Desinfektion durchzuführen,
**dass** jede Sonde (7) Identifikationsinformationen (7c) aufweist,
und **dass** die Kammer (11) folgenden Komponenten zugeordnet ist: Einrichtungen (16) zum Erfassen der Identifikationsinformationen der bzw. jeder Sonde (7) bei deren Anordnen in der Kammer (11) und bei deren Entnahme aus der Kammer (11) zu Beginn bzw. am Ende eines Desinfektionszyklus, Einrichtungen (17, 18) zum Erfassen von Kennzeichnungsinformationen des Desinfektionszyklus, und Einrichtungen (19, 20, 21, 22, 23) zum Zuordnen der Identifikationsinformationen der bzw. jeder Sonde und der Kennzeichnungsinformationen des Desinfektionszyklus, um Informationen für die Rückverfolgbarkeit der Desinfektion der bzw. jeder Sonde zu erzeugen.

2. Vorrichtung nach Anspruch 1,
**dadurch gekennzeichnet,**
**dass** die Kammer mit einer Zugangsklappe (12) ausgestattet ist und einem an dem Gestell (2) vorgesehenen Steuerpult (14) zugeordnet ist.

3. Vorrichtung nach Anspruch 1 oder 2,
**dadurch gekennzeichnet,**
**dass** die Identifikationsinformationen (7c) jeder Sonde in Form eines Strichcodes vorliegen,
und **dass** die entsprechenden Erfassungseinrichtungen (16) der Kammer (11) ein Codelesegerät aufweisen.

4. Vorrichtung nach Anspruch 3,
**dadurch gekennzeichnet,**
**dass** die Kammer (11) einen Ausleger (15) zum Aufhängen der Sonde aufweist,
und **dass** das Lesegerät (16) an diesem Ausleger befestigt ist.

5. Vorrichtung nach einem der Ansprüche 1 bis 4,
**dadurch gekennzeichnet,**
**dass** die Einrichtungen (17, 18) zum Erfassen von Kennzeichnungsinformationen des Desinfektionszyklus Einrichtungen zum Erfassen von Informationen aufweisen, die aus der Gruppe von Informationen ausgewählt sind, welche Identifikationsinformationen der Kammer (11) und/oder der Vorrichtung und Zeitmarkierungsinformationen des Desinfektionszyklus umfasst.

6. Vorrichtung nach einem der Ansprüche 1 bis 5,
**dadurch gekennzeichnet,**
**dass** die Kammer (11) Einrichtungen zum Erzeugen von UV-Strahlung zum Desinfizieren der Sonde aufweist,
und **dass** die Kennzeichnungsinformationen des Desinfektionszyklus Informationen über die während des Zyklus abgegebene UV-Strahlungsdosis umfassen, die von einem entsprechenden, in der Kammer angebrachten Sensor (18) stammen.

7. Vorrichtung nach den Ansprüchen 4 oder 5 und 6,
**dadurch gekennzeichnet,**
**dass** der UV-Sensor (18) unter dem Ausleger der Kammer angebracht ist.

8. Vorrichtung nach einem der Ansprüche 1 bis 7,
**dadurch gekennzeichnet,**
**dass** die Einrichtungen (19) zum Zuordnen der Informationen Einrichtungen für deren Visualisierung (22), Speicherung (21) und/oder Ausdrucken (23) zugeordnet sind.

9. Vorrichtung nach einem der Ansprüche 1 bis 8,
**dadurch gekennzeichnet,**
**dass** die Einrichtungen (19) zum Zuordnen der Informationen dazu ausgelegt sind, die Rückverfolgbarkeitsinformationen nur dann auszugeben, wenn die entsprechende Sonde (7) bei deren Anordnen in der Kammer (11) und deren Entnahme aus der Kammer (11) vor bzw. nach dem Desinfektionszyklus eindeutig identifiziert worden ist.

10. Vorrichtung nach einem der vorherigen Ansprüche,
**dadurch gekennzeichnet,**
**dass** die Kammer (11) eine Innenfläche (30) mit einem kreisförmigen Querschnitt aufweist.

11. Vorrichtung nach Anspruch 10,
**dadurch gekennzeichnet,**
**dass** die Innenfläche (30) der Kammer halbkreisförmige Vertiefungen (36, 37, 38, 39) zum Aufnehmen der Einrichtungen (31, 32, 33, 34) zum Erzeugen der Strahlung aufweist.

12. Vorrichtung nach Anspruch 11,
**dadurch gekennzeichnet,**
**dass** die Einrichtungen zum Erzeugen der Strahlung vier Röhren aufweisen, die jeweils um 90° voneinander beabstandet in der Kammer angeordnet sind.

13. Vorrichtung nach einem der vorherigen Ansprüche,
**dadurch gekennzeichnet,**
**dass** die Kammer (11) eine Innenfläche aufweist, die zumindest teilweise mit Polytetrafluorethylen (PTFE) beschichtet ist.

14. Vorrichtung nach einem der vorherigen Ansprüche,
**dadurch gekennzeichnet,**
**dass** die Kammer (11) Einrichtungen zum Aufhängen von mindestens einer Sonde in der Kammer und Einrichtungen (40, 41) zum Drehen der Sonde (7) in der Kammer aufweist.

15. Vorrichtung nach Anspruch 14,
**dadurch gekennzeichnet,**
**dass** die Dreheinrichtungen dazu ausgelegt sind, eine Oszillationsbewegung der Aufhängungseinrichtungen über einen vorgegebenen Winkelbereich hervorzurufen.

16. Vorrichtung nach Anspruch 14 oder 15,
**dadurch gekennzeichnet,**
**dass** die Dreheinrichtungen elektrische Antriebseinrichtungen (41) aufweisen, die einem Haken (40) zum Aufhängen der Sonde (7) in der Kammer zugeordnet sind.

17. Vorrichtung nach den Ansprüchen 4 und 16,
**dadurch gekennzeichnet,**
**dass** die Aufhängungseinrichtungen und die Antriebseinrichtungen dem Ausleger (15) zugeordnet sind, der sich in der Kammer erstreckt.

18. Vorrichtung nach einem der vorherigen Ansprüche,
**dadurch gekennzeichnet,**
**dass** die Kammer Einrichtungen (15) zum Aufhängen von mindestens einer Sonde (7) in der Kammer und Einrichtungen zum Erzeugen einer Desinfektionsstrahlung in der Kammer sowie für die Strahlung durchlässige Einrichtungen (50) aufweist, welche die Sonde (7) in einem langgestreckten Zustand und einer im wesentlichen vertikalen Ausrichtung unter den Aufhängungseinrichtungen (15) halten.

19. Vorrichtung nach Anspruch 18,
**dadurch gekennzeichnet,**
**dass** die Halteeinrichtungen dazu ausgelegt sind, die Sonde (7) im wesentlichen in der Mitte der Kammer (11) zu halten.

20. Vorrichtung nach Anspruch 18 oder 19,
**dadurch gekennzeichnet,**
**dass** die Halteeinrichtungen Einrichtungen (51, 52, 53, 54) zum Führen der Sonde in der Kammer aufweisen.

21. Vorrichtung nach Anspruch 20,
**dadurch gekennzeichnet,**
**dass** die Führungseinrichtungen ein oder mehrere kammartige Organe (51, 52, 53, 54) zum Führen der Sonde aufweisen, die über die Höhe der Kammer (11) verteilt sind.

22. Vorrichtung nach Anspruch 21,
**dadurch gekennzeichnet,**
**dass** die Führungseinrichtungen eine oder mehrere Röhren zum Aufnehmen der Sonde (7) aufweisen.

23. Vorrichtung nach einem der Ansprüche 18 bis 22,
**dadurch gekennzeichnet,**
**dass** die Halteeinrichtungen ein Gewicht aufweisen, das dazu ausgelegt ist, an dem freien Ende der Sonde (7) eingehängt zu werden.

24. Vorrichtung nach einem der Ansprüche 18 bis 23,
**dadurch gekennzeichnet,**
**dass** die Halteeinrichtungen aus Siliciumoxid hergestellt sind.
